Europäisches Patentamt

European Patent Office

Office européen des brevets

(11) Veröffentlichungsnummer: **0 016 178**
**B1**

(12) **EUROPÄISCHE PATENTSCHRIFT**

(45) Veröffentlichungstag der Patentschrift:
**29.09.82**

(21) Anmeldenummer: **79900949.3**

(22) Anmeldetag: **21.08.79**

(86) Internationale Anmeldenummer:
**PCT/DE 79/00093**

(87) Internationale Veröffentlichungsnummer:
**WO 80/00442 (20.03.80 Gazette 80/6)**

(51) Int. Cl.³: **C 07 C 9/04**, C 07 C 1/00,
C 10 J 3/10

(54) **Anlage zur Kohlevergasung.**

(30) Priorität: **31.08.78 DE 2837288**

(43) Veröffentlichungstag der Anmeldung:
**01.10.80 Patentblatt 80/20**

(45) Bekanntmachung des Hinweises auf die Patenterteilung:
**29.09.82 Patentblatt 82/39**

(84) Benannte Vertragsstaaten:
**AT CH DE FR GB LU NL SE**

(56) Entgegenhaltungen:
**DE-A-2 553 506**
**DE-A-2 704 465**

**I. Spiewak et al.: «Assessment of Very-High Temperature in Process Applications» ORNL/TM-5242, veröffentlicht im November 1976, Oak Ridge National Laboratory, Oak Ridge, Siehe Seite 82, Figur 24**

(73) Patentinhaber: **GHT Gesellschaft für Hochtemperaturreaktor-Technik mbH, c/o Siemens AG Postfach 261, D-8000 München 22 (DE)**

(72) · Erfinder: **FISCHER, Reimer, Lückerather Weg 71, D-5060 Bergisch Gladbach 2 (DE)**
Erfinder: **JÄGER, Walter, Schulweg 33, D-5250 Engelskirchen (DE)**
Erfinder: **VON WACLAWICZEK, Herbert, Friedrich-Offermann Strasse 59, D 5060 Bergisch Gladbach (DE)**

(74) Vertreter: **Mehl, Ernst, Dipl.-Ing., Postfach 22 01 76, D-8000 München 22 (DE)**

Anlage zur Kohlevergasung

Die vorliegende Erfindung betrifft eine Anlage zur Erzeugung von Methan oder Synthesegas aus kohlenstoffhaltigen Stoffen ohne Verbrennung dieser Stoffe, insbesondere unter Ausnutzung der Kernenergie. Dabei wird ein erster Teil des Kohlenstoffs mit Wasserstoff in einem hydrierenden Vergaser zu Methan umgesetzt und ein zweiter Teil des Kohlenstoffs mit Wasserdampf in einem Wasserdampfvergaser zu Synthesegas umgesetzt; Zumindest ein Teil des Methans wird mit Wasserdampf unter Wärmezufuhr zu Synthesegas umgesetzt. Diese Anlagen liefern entweder Methan ($CH_4$) oder Synthesegas ($H_2$ und $CO$) oder beide Gase in jedem gewünschten Verhältnis.

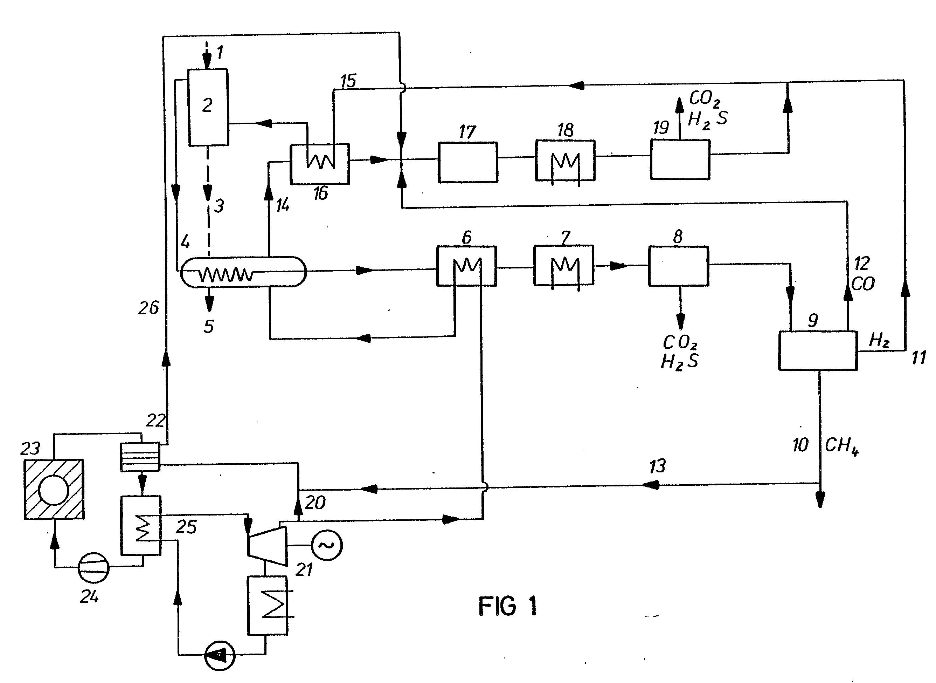

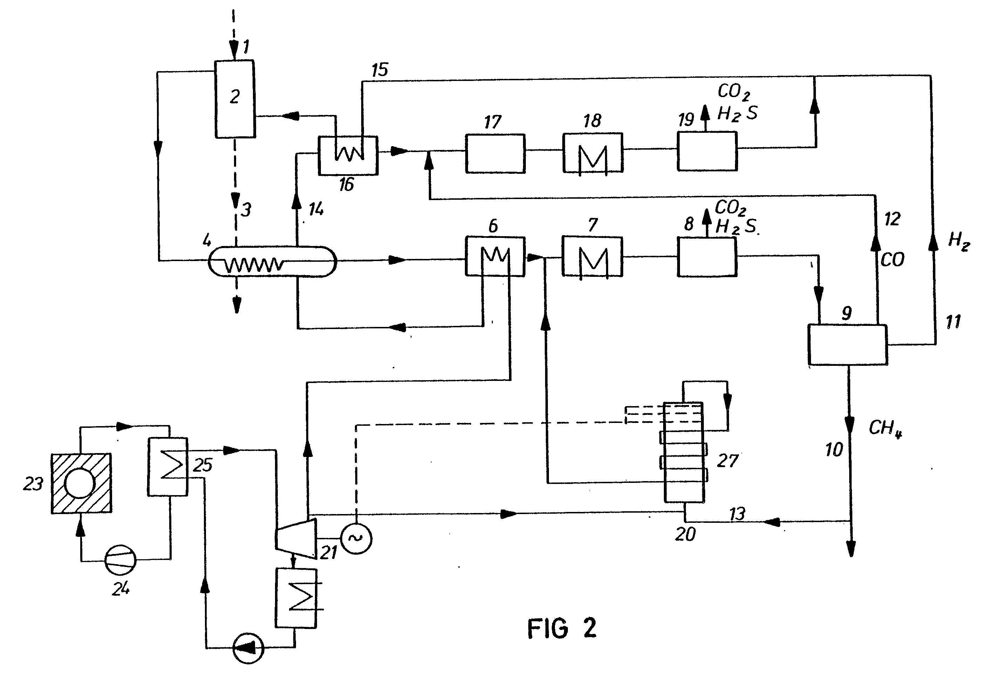

In der Zeitschrift: «Chemie-Ingenieur-Technik» 1974 wird auf Seite 938, insbesondere in Abbildung 1, sowie auf Seite 941, insbesondere in Abbildung 2 je ein Prozessschema beschrieben zur Herstellung von Methan über die Wasserdampfvergasung von Kohle. Auf Seite 937 werden neue Verfahren einer hydrierenden Vergasung von Kohle zu Methan erwähnt. Beide Verfahren sind aber mit einem erheblichen Nachteil behaftet. Bei der hydrierenden Versagung ist wegen der grossen Verweilzeiten der Kohle und mit Rücksicht auf die begrenzten Abmessungen des Vergasers keine vollkommene Umsetzung der Kohle erreichbar. Der bei der hydrierenden Vergasung abfallende Restkoks enthält ausser der Asche noch ca. 30-45% des eingesetzten Kohlenstoffs. Bei der Wasserdampfvergasung dagegen kann man zwar den eingesetzten Kohlenstoff nahezu restlos vergasen. Da dieser Prozess aber nur bei hohen Temperaturen abläuft (Steinkohle 790°C, Braunkohle 630-660°C), ist nur der obere Temperaturbereich der im Reaktor freiwerdenden Wärme für die Vergasung auszunutzen. Die restliche Wärme kann bei einem reinen Wasserdampfvergaser-Prozess im wesentlichen nur noch zur Dampferzeugung und damit zur Stromerzeugung benutzt werden, weil nur ein kleiner Teil dieses Dampfes im Prozess verwendet werden kann.

In dem Bericht ORNL/TM-5242 (Oak Ridge National Laboratory, November 1976) wird auf Seite 82 eine Anlage dargestellt, in der mittels nuklearer Wärme Kohlenstoff in Methan umgesetzt wird. Die Kohle wird zunächst getrocknet, dann hydrierend vergast und der Restkoks in einem Wasserdampfvergaser zu Synthesegas umgewandelt. Ein Teil des produzierten Methans wird in einem Methanspaltofen (dort als Reformer bezeichnet) unter Zusatz von heissem Wasserdampf zu Synthesegas umgesetzt. Diese Schaltung hat aber noch folgende Nachteile: Die in einem primären aber auch in einem sekundären Heliumkreis angeordneten Prozesswärmetauscher sind sehr aufwendig. Einerseits stellen Helium führende Rohrleitungen und Apparate erhöhte Anforderungen an die Dichtigkeit und andererseits muss auch verhindert werden, dass unerwünschte Stoffe auf diese Weise in den Primärkreis eindringen und dort entweder korrodierend wirken, unerwünschte Ablagerungen bilden oder derart aktiviert werden, dass sie an anderer Stelle stören. Ausserdem haben alle mit reinem Helium beheizten Prozesswärmetauscher den Nachteil, dass Helium als einatomiges Gas keine Strahlungswärme abgibt, was besonders bei den hier vorgesehenen hohen Temperaturen von Nachteil ist. Man müsste also die Heliumgeschwindigkeit erhöhen, um den Wärmeübergang durch Konvektion zu erhöhen, was den Druckverlust erhöht, oder die Heizflächen vergrössern.

In den deutschen Offenlegungsschrift Nr. 25 53 506.2 wird eine Anlage zur Erzeugung von Methan oder Synthesegas aus kohlenstoffhaltigen Stoffen mit Hilfe eines Kernreaktors beschrieben, wobei ein Teil des kohlenstoffhaltigen Stoffes unter Zugabe von Wasserdampf und bei hoher Temperatur in einem Wasserdampfvergaser zu Synthesegas umgewandelt wird. Diese Anlage hat zwei Reaktorkühlkreise, wobei in einem ersten Kühlkreis der Wasserdampfvergaser angeordnet ist und in einem zweiten Kühlkreis ein an sich bekannter Spaltofen angeordnet ist, in dem ein Teil des erzeugten Methans bei hoher Temperatur zu Wasserstoff gespalten wird, der zur hydrierenden Vergasung eines anderen Teils des kohlenstoffhaltigen Stoffes benutzt wird. Diese zweistufige Vergasung verbindet in vorteilhafter Weise die Anforderungen: niedriger Kohleverbrauch, vollständige Vergasung der Kohle, Einbindung nahezu der gesamten Kernreaktorwärme in den Vergasungsprozess, so dass fast kein Strom nach aussen abgegeben werden muss, und hoher Gesamtwirkungsgrad. Nachteilig ist dabei noch, dass für den Wasserdampfvergaser die notwendige Beheizung bei hoher Temperatur entweder direkt oder über einen Zwischenkreislauf vom nuklear beheizten Helium bereitgestellt werden muss. Dieses Helium müsste eine Temperatur von ca. 900-950°C haben und die Anlagenteile zwischen Kernreaktor und Vergasungsanlage müssen für diese hohe Temperatur geeignet sein. Der Helium-Sekundärkreislauf, der die Wärme des Kernreaktors von dessen Primärkreislauf auf die Vergasungsanlage überträgt, ist mit einem erheblichen Aufwand und auch mit zusätzlichen Verlusten verbunden. Die enge Kopplung zwischen Kernreaktor- und Vergasungsanlage bedingt einen erheblichen sicherheitstechnischen Aufwand und erschwert den Betrieb der Gesamtanlage.

Aufgabe der vorliegenden Erfindung ist eine Anlage zur Erzeugung von Methan oder Synthesegas aus kohlenstoffhaltigen Stoffen ohne Verbrennung dieser Stoffe; Die Anlage soll die beschriebenen Nachteile weitgehend vermeiden und von einer Energieversorgungsanlage weitgehend entkoppelt sein, so dass Störungen an der einen Anlage sich nicht unmittelbar auf die andere Anlage auswirken können.

Zur Lösung dieser Aufgabe wird eine Anlage nach dem 1. Anspruch vorgeschlagen. Die gegenseitige Beheizung der beiden Vergaser erspart einen in der bekannten Anlage dargestellten zweiten Heizkreislauf und zwar aus folgendem Grund:

Durch die exotherme Reaktion der hydrierenden Vergasung ist es möglich, ohne Wärmezufuhr von aussen diese Vergasungsstufe auf höherem Temperaturniveau als die folgende Wasserdampfvergasung ablaufen zu lassen und damit das Rohgas von der hydrierenden Vergasung zur indirekten Beheizung der endothermen Wasserdampfvergasung zu benutzen. Bei der bekannten Anlage muss an dieser Stelle Hochtemperaturwärme eines Kernreaktors eingebracht werden. Da das Rohgas aus dem hydrierenden Vergaser mit einer sehr hohen Temperatur austritt und es unwirtschaftlich ist, diese hohe Temperatur wie bisher nur zur Vorwärmung des eintretenden Rohgases oder nur zur Dampferzeugung auszunutzen, ist mit dieser Schaltung ein guter Gesamtwirkungsgrad der Anlage zu erwarten. Die Anforderungen an die Dichtigkeit eines Wärmetauschers, insbesondere im Bereich des Wasserdampfvergasers, sind erheblich geringer, wenn auf beiden Seiten nur Prozessgase geführt werden und nicht ein Reaktorkühlmedium. Der im Rohgas enthaltene Staub kann beispielsweise wie im deutschen Patent 12 44 120 beschrieben, abgeschieden werden. Der wesentlichste Vorteil dieser Anlage ist aber, dass die Vergasungsanlage weitgehend von ihrer Energieversorgung, insbesondere einer Kernreaktoranlage, entkoppelt ist. Kernenergieanlagen werden aus Sicherheitsgründen in einem sogenannten Sicherheitsbehälter angeordnet, der alle radioaktiven Kreisläufe umschliesst, und der bei einem Schadensfall vollständig verschlossen werden kann. Dieser Sicherheitsbehälter kann von Leitungen für Wasser bzw. Dampf oder auch für Gas durchdrungen werden. Die kontinuierliche Beschickung mit grossen Mengen von Feststoffen und die Ausschleusung des Restkokses oder der Asche ist aber mit einem erheblichen Aufwand verbunden. Ausserdem kann der Vergaser unabhängig vom Reaktor mit dem günstigsten Betriebsdruck, z.B. 80 bar betrieben werden.

Die Anlage nach dem 2. Anspruch hat als einzige Kopplung zwischen Vergasungsanlage und Kernreaktor einen Methanspaltofen, der mit geringem Aufwand innerhalb des Sicherheitsbehälters angeordnet werden kann und auch ohne einen Sekundärkühlkreis unmittelbar vom Primärhelium beheizt werden kann. Diesem Methanspaltofen sollte in üblicher Weise ein Rekuperativ-Wärmetauscher vorgeschaltet sein, der einen grossen Teil der Wärme des austretenden Synthesegases auf das eintretende Methan überträgt, so dass die den Sicherheitsbehälter durchdringenden Gasleitungen nur geringe Temperaturen aufweisen und die Vergasungsanlage mit sicherheitstechnisch ausreichendem Abstand vom Reaktor aufgestellt werden kann.

Die Anlage nach dem 3. Anspruch hat eine noch weitergehende Trennung zwischen Energieversorgung und Vergasung. Wenn man den Methanspaltofen elektrisch beheizt, kann man den Kernreaktor, der sowohl ein Hochtemperaturreaktor als auch ein Druckwasserreaktor sein kann, noch mehr von der Vergaseranlage trennen. Als einzige Leitung, die den Sicherheitsbehälter des Kernreaktors durchdringen müssen, sind nur noch Wasser- bzw. Dampfleitungen vorhanden, deren Technologie bei Kernreaktoranlagen inzwischen zum gesicherten Stand der Technik gehört. Die Energieübertragung vom Kernreaktor über Dampferzeuger, Turbine und Gnerator bis zur elektrischen Beheizung ist naturgemäss gegenüber der direkten Beheizung mit einem schlechteren Wirkungsgrad verbunden. Da aber nur ein Teil der Kernenergie zur Stromerzeugung verwendet wird und ein grosser Teil der Kernenergie ohne wesentlichen Verlust als Prozessdampf in die Vergasungsanlage eingeführt wird, hat die Gasamtanlage einen tragbaren Wirkungsgrad bei einem wesentlich geringeren Investitionsaufwand. Unter diesen Umständen kann man sogar die Kernenergie beispielsweise mit einem Druckwasserreaktor bereitstellen. Für Prototyp- und Versuchsanlagen oder auch für kleinere Vergasungsanlagen kann es sogar sinnvoll sein, die für Strom- und Prozessdampfversorgung notwendige Energie zum Teil durch konventionelle Verbrennung, beispielsweise von minderwertigen Brennstoffen bereitzustellen.

Die Fig. 1 zeigt ein stark schematisiertes Beispiel der Erfindung. Die gemahlene und getrocknete Kohle wird bei 1 dem hydrierenden Vergaser 2 zugeführt und dort exothermisch hydrierend vergast. Der dort abfallende Restkoks wird auf dem Weg 3 dem Wasserdampfvergaser 4 zugeführt und weiter vergast. Der dort abfallende Rückstand besteht aus Asche mit einem geringen Anteil Kohlenstoff und wird bei 5 abgezogen. Das aus dem hydrierenden Vergaser 2 austretende Rohgas gibt einen Teil seiner Wärme im oberen Temperaturbereich im Wasserdampfvergaser 4 ab und im unteren Temperaturbereich im Prozessdampfüberhitzer 6 und im Dampferzeuger 7. Nach Abkühlung und Entfernung von Staub und Teer wird das Rohgas in einer Gaswäsche 8 von Kohlendioxyd und Schwefelwasserstoff befreit. In der Tieftemperaturzerlegung 9 wird das gereinigte Gas zerlegt in eine Methan-Fraktion 10, in eine Wasserstoff-Fraktion 11 und eine Kohlenmonoxyd-Fraktion 12. Das Methan wird teilweise einem Gasnetz und teilweise auf dem Wege 13 einem Spaltofen zugeführt. Das den Wasserdampfvergaser 4 auf dem Wege 14 verlassende Hydriergas gibt einen Teil seiner Wärme an das dem hydrierenden Vergaser 2 zuzuführende Hydriergas 15 in dem Wärmetauscher 16 ab. Das im Hydriergas enthaltene Kohlenmonoxyd wird in der Konvertierung 17 mit Wasserdampf in Wasserstoff und Kohlendioxyd überführt. Danach wird das Hydriergas in dem Abhitzekessel 18 weiter abgekühlt, wobei Prozessdampf erzeugt wird. Nachdem in der Gaswäsche 19 Kohlendioxyd und Schwefelwasserstoff entfernt wurden, wird das derart gereinigte Gas mit dem aus der Tieftemperaturzerlegung 9 abgegebenen Wasserstoff auf dem Wege 15 im Wärmetauscher 16 vorgewärmt und dem hydrierenden Vergaser 2 zugeleitet, wo es einen grossen Teil des Kohlenstoffs in Methan verwandelt. Der aus der Tieftemperaturgaszerlegung 9 austretende Methanstrom 13 wird bei 20 mit Wasserdampf aus der Dampfkraftanlage 21 vermischt und im heliumbeheizten Röhrenspaltofen 22 zu Kohlenmonoxyd und Wasserstoff mit einem geringen Anteil von Methan und Wasserdampf gespalten. Die dafür notwendige Wärmeenergie wird von einem Hochtemperaturreaktor 23 bereitgestellt, dessen geschlossener Heliumkreislauf mit einem Gebläse 24 betrieben wird und der die im Spaltofen 22 nicht verwertbare Wärme an einen Dampferzeuger 25 abgibt. Das aus dem Spaltofen 22 austretende Hydriergas hat einen grossen Teil seiner Wärme rekuperativ an das eintretende Methan abgegeben und wird auf dem Wege 26 und zwar vor der Konvertierung 17 dem aus dem Wasserdampfvergaser 4 austretenden Hydriergas zugemischt.

Fig. 2 zeigt ein weiteres stark schematisiertes Beispiel der Erfindung. Gegenüber der in Fig. 1 bereits beschriebenen Anlage ist hier anstelle des mit Helium beheizten Methanspaltofens 22 ein Methanspaltofen 27 vorhanden, der in seinem oberen Temperaturbereich mit elektrischem Strom aus der Dampfturbinenanlage 21 beheizt wird und der bei 20 mit einem Gemisch aus Methan und Wasserdampf beschickt wird. Das austretende gespaltene Rohgas dient im Kreuzgegenstrom zur Beheizung des eintretenden Methan-Wasserdampfgemisches und wird dem aus dem Wasserdampfvergaser austretenden Rohgas zwischen dem Prozessdampferhitzer 6 und dem Prozessdampferzeuger 7 zugeführt. Diese Anlage ist nicht auf einen gasgekühlten Hochtemperaturreaktor angewiesen und kann daher auch von einem Leichtwasserreaktor über einen Dampfkreislauf mit Energie versorgt werden. Da ein grosser Teil des nuklear beheizten Wasserdampfes direkt in der Anlage verwertet wird, wird der geringere thermische Wirkungsgrad des Leichtwasserreaktors, insbesondere bei kleineren Anlagen, durch die geringeren Investitionskosten etwas ausgeglichen.

Die folgende Zahlenbeispiele beziehen sich auf Kohlevergasungsanlagen, die mit einem Hochtemperaturreaktor oder einem Leichtwasserreaktor beheizt werden.

Thermische Reaktorleistung:      1 500 MW
Kohleart: Gasflammkohle mit 38% flüchtigen
          Bestandteilen

Beispiel 1
Erzeugung von SNG
Kohledurchsatz:      531   t/h

Produkt:      381 · 10³   m³$_N$/h
Brennwert:      40 693   KJ/m³$_N$
Zusammensetzung: 95% CH$_4$ und 5% C$_2$H$_6$

Beispiel 2
Erzeugung von Synthesegas
Kohledurchsatz:      433   t/h
Produkt:      1 249   m³$_N$/h
Brennwert:      8 673   KJ/m³$_N$
Zusammensetzung: 68% H$_2$ und 32% CO

**Patentansprüche**

1. Anlage zur Erzeugung von Methan oder Synthesegas aus kohlenstoffhaltigen Stoffen ohne Verbrennung dieser Stoffe mit einem hydrierenden Vergaser (2), in dem ein erster Teil des Kohlenstoffs mit Wasserstoff zu Methan umgesetzt wird; einem Wasserdampfvergaser (4) in dem ein weiter Teil des Kohlenstoffs mit Wasserdampf zu Synthesegas umgesetzt wird und einem Spaltofen (22) oder (27), in dem zumindest ein Teil des Methans mit Wasserdampf zu Synthesegas umgesetzt wird, gekennzeichnet durch folgende Merkmale:
a) Wärmetauschkanäle im Wasserdampfvergaser (4), in denen das aus dem hydrierenden Vergaser (2) austretende Rohgas Wärme abgibt.
b) Einem Wärmetauscher (16), in dem das aus dem Wasserdampfvergaser (4) strömende Rohgas Wärme an das in den hydrierenden Vergaser (2) eintretende Wasserstoffgas abgibt.

2. Anlage nach Anspruch 1 zur Erzeugung von Synthesegas mit einem heliumgekühlten Hochtemperaturreaktor (23), gekennzeichnet durch folgendes Merkmal:
a) Im Kühlkreislauf des Kernreaktors sind nacheinander ein Methanspaltofen (22) und ein Dampferzeuger (25) angeordnet.

3. Anlage nach Anspruch 1 mit einem Kernreaktor (23), in dessen Kühlkreislauf ein Dampferzeuger (25) zur Versorgung eines Turbogenerators (21) angeordnet ist, gekennzeichnet durch folgende Merkmale:
a) Der Methanspaltofen (27) ist mit einer elektrischen Zusatzheizung versehen.
b) Die elektrische Zusatzheizung ist an den Turbogenerator (21) angeschlossen.

**Claims**

1. An istallation for the production of methane or synthesis gas from substances containing carbon without combustion of these substances, having a hydrogenating gasifier (2) in which a first part of the carbon is reacted with hydrogen to form methane; a steam gasifier (4) in which a second part of the carbon is reacted with steam to form synthesis gas; and a cracking furnace (22) or (27), in which at least a part of the methane is reacted with steam to form synthesis gas, characterised by the following features:

a) heat exchange channels in the steam gasifier (4) in which the crude gas leaving the hydrogenating gasifier (2) gives úp heat;

b) a heat exchanger (16) in wich the crude gas flowing from the steam gasifier (4) gives up heat to the inlet hydrogen gas to the hydrogenating gasifier (2).

2. An istallation as claimed in Claim 1 for the production of synthesis gas, having a helium-cooled high temperature reactor (23), characterised by the following feature:

a) a methane cracking furnace (22) and a steam generator (25) are arranged in series in the cooling circuit of the nuclear reactor.

3. An installation as claimed in Claim 1 having a nuclear reactor (23) the cooling circuit of which includes a steam generator (25) which serves to supply a turbo-generator (21) characterised by the following features:

a) the methane cracking furnace (27) is provided with an auxiliary electrical heating unit;

b) the auxiliary electrical heating unit is connected to the turbo-generator (21).

**Revendications**

1. Installation pour obtenir du méthane ou du gaz de synthèse à partir de substances carbonées sans combustion de ces substances, comprenant un gazéificateur (2) par hydrogénation dans lequel une première partie du carbone est transformée en méthane par de l'hydrogène; un gazéificateur (4) à la vapeur d'eau dans lequel une seconde partie du carbone est transformée en gaz de synthèse par de la vapeur d'eau et un four (22 ou 27) de dissociation dans lequel au moins une partie de méthane est transformée en gaz de synthèse par de la vapeur d'eau, caractérisée par les caractéristiques suivantes:

a) des canaux d'échange de chaleur dans le gazéificateur (4) à la capeur d'eau dans lesquels le gaz brut sortant du gazéificateur (2) par hydrogénation (7) cède de la chaleur;

b) un échangeur (16) de chaleur dans lequel le gaz brut sortant du gazéificateur (4) à la vapeur d'eau cède de la chaleur à l'hydrogène gazeux entrant dans le gazéificateur (2) par hydrogénation.

2. Installation suivant la revendication 1, pour l'obtention de gaz de synthèse à l'aide d'un réacteur (23) à haute température refroidi par de l'hélium, caractérisée par la caractéristique suivante:

a) dans le circuit de refroidissement du réacteur nucléaire sont montés, l'un après l'autre, un four (22) de dissociation du méthane et un générateur (25) de vapeur.

3. Installation suivant la revendication 1, comprenant un réacteur (23) nucléaire dans le circuit de refroidissement duquel est monté un générateur (25) de vapeur destiné à l'alimentation d'un turbogénérateur (21), caractérisée par les caractéristiques suivantes:

a) le four (27) de dissociation du méthane est pourvu d'un chauffage électrique supplémentaire;

b) le chauffage électrique supplémentaire est relié au tourgénérateur (21).

FIG 1

FIG 2